(19) Europäisches Patentamt
European Patent Office
Office européen des brevets

(11) **EP 3 130 273 B1**

(12) **EUROPEAN PATENT SPECIFICATION**

(45) Date of publication and mention
of the grant of the patent:
**15.05.2019 Bulletin 2019/20**

(51) Int Cl.:
***A61B 1/00*** *(2006.01)*     ***G06T 7/00*** *(2017.01)*

(21) Application number: **15180882.1**

(22) Date of filing: **13.08.2015**

(54) **STEREOSCOPIC VISUALIZATION SYSTEM AND METHOD FOR ENDOSCOPE USING SHAPE-FROM-SHADING ALGORITHM**

STEREOSKOPISCHES VISUALISIERUNGSSYSTEM UND VERFAHREN FÜR ENDOSKOPE MIT SHAPE-FROM-SHADING-ALGORITHMUS

SYSTÈME ET PROCÉDÉ DE VISUALISATION STÉRÉOSCOPIQUE POUR ENDOSCOPE UTILISANT UN ALGORITHME PAR OMBRAGE DE FORME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**15.02.2017 Bulletin 2017/07**

(73) Proprietor: **MedicalTek Co., Ltd.**
**Taichung City 42881 (TW)**

(72) Inventors:
• **KUMAR, Atul**
**Changhua County 505 (TW)**
• **WANG, Yen-Yu**
**Nantou County 542 (TW)**
• **LIU, Kai Che**
**Kaohsiung City 807 (TW)**
• **WANG, Min-Liang**
**Taichung City 43858 (TW)**

(74) Representative: **advotec.**
**Patent- und Rechtsanwälte**
**Beethovenstrasse 5**
**97080 Würzburg (DE)**

(56) References cited:
**WO-A1-2012/075631     WO-A1-2014/027229
US-A1- 2014 198 976**

• **MARCO VISENTINI-SCARZANELLA ET AL: "Metric depth recovery from monocular images using Shape-from-Shading and specularities", IMAGE PROCESSING (ICIP), 2012 19TH IEEE INTERNATIONAL CONFERENCE ON, IEEE, 30 September 2012 (2012-09-30), pages 25-28, XP032333108, DOI: 10.1109/ICIP.2012.6466786 ISBN: 978-1-4673-2534-9**

**Description**

BACKGROUND OF THE INVENTION

Field of the Invention

The present invention relates to a stereoscopic visualization system for endoscope and, more particularly, to a stereoscopic visualization system for endoscope using shape-from-shading algorithm to generate stereo images.

Description of Related Art

Minimally invasive surgery has become an indispensable part in surgical treatment of current medical behavior and can be performed by endoscope-assisted surgical instruments to allow smaller incision and less tissue trauma, thereby shortening patient's recovery cycle and reducing overall medical expense. However, conventional minimally invasive surgery all employs monoscopic endoscope, which only displays two-dimensional (2D) images lacking depth information. Therefore, it is challenging for a surgeon to accurately move surgical instruments to a correct location inside a patient's body. Surgeons usually perceive depth in 2D images according to motion parallax, monocular cues and other indirect evidences for positioning accuracy. Providing stereo images capable of directly providing depth perception without going through additional means, such as motion parallax, monocular cues and other indirect evidences, is still the best approach in resolving the conventional inaccurate positioning issue at the cost of a dual-camera endoscope. Despite the advantages of depth information or stereo images required by surgeons, the dual-camera endoscope has the drawback of being much more expensive than the monoscopic endoscope and is less accepted accordingly. The prior art document US-A-2014/198976 provides a stochastic method and system for fast stereoscopic ranging includes selecting a pair of images for stereo processing.

SUMMARY OF THE INVENTION

An objective of the present invention is to provide a stereoscopic visualization system and a stereoscopic visualization method using shape-from-shading algorithm capable of providing stereoscopic images with a monoscopic endoscope through the shape-from-shading algorithm.

To achieve the foregoing objective, the stereoscopic visualization system for endoscope using shape-from-shading algorithm includes a monoscopic endoscope, a three-dimensional (3D) display, and an image conversion device.

The monoscopic endoscope is configured to capture the two-dimensional (2D) images.

The image conversion device may be connected between the monoscopic endoscope and the 3D display and may have an input port for endoscope and a 2D-to-3D conversion unit.

The input port for endoscope is connected to the monoscopic endoscope to receive the 2D image from the monoscopic endoscope.

The 2D-to-3D conversion unit is configured to apply shape from shading algorithm adapted to calculate a direction of a light source for the 2D image, and to calculate a depth map based upon information of light distribution and shading of the 2D image, and to apply depth image based rendering algorithm to convert the 2D image to a stereoscopic image with the information of light distribution and shading of the 2D image.

The image output port is connected with the 2D-to-3D image conversion unit and the 3D display to receive the stereo images and display the stereo image on the 3D display.

To achieve the foregoing objective, the stereoscopic visualization method for endoscope using shape-from-shading algorithm includes steps as defined by claim 2.

Given the foregoing stereoscopic visualization system and method using shape-from-shading method, the 2D image taken by the monoscopic endoscope is processed by the shape-from-shading algorithm to calculate depth information in generation of a depth map, and the 2D image along with the depth map form the stereoscopic image that is outputted to the 3D display for users to view the converted stereoscopic image. As there is no need to replace a monoscopic endoscope with a dual-lens endoscope and modify the hardware structure of the existing monoscopic endoscope, the issues of no stereoscopic image available to monoscopic endoscope and costly dual-lens endoscope encountered upon the demand of stereoscopic images can be resolved.

Other objectives, advantages and novel features of the invention will become more apparent from the following detailed description when taken in conjunction with the accompanying drawings.

BRIEF DESCRIPTION OF THE DRAWINGS

FIG. 1 is a functional block diagram of a stereoscopic visualization system for endoscope using shape-from-shading algorithm in accordance with the present invention.

FIG. 2 is a flow diagram of a stereoscopic visualization method for endoscope using shape-from-shading algorithm in accordance with the present invention.

DETAILED DESCRIPTION OF THE PREFERRED EMBODIMENTS

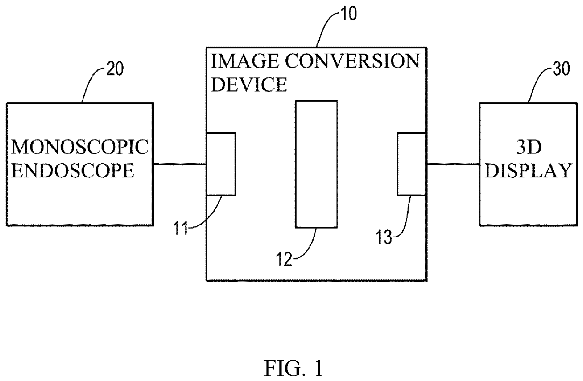

With reference to FIG. 1, a stereoscopic visualization system for endoscope using shape-from-shading algorithm in accordance with the present invention includes a monoscopic endoscope 20, a three-dimensional (3D) display 30, and an image conversion device 10.

The image conversion device 10 is connected between the monoscopic endoscope 20 and the 3D display 30, and has an input port for endoscope 11, a 2D-to-3D image conversion unit 12, and an image output port 13. The input port for endoscope 11 is connected to the monoscopic endoscope 20. The 2D-to-3D image conversion unit 12 is electrically connected to the input port for endoscope 11, acquires a 2D image from the monoscopic endoscope 20, generates a depth map of the 2D image, and converts the 2D images and the depth map into a stereoscopic image using shape-from-shading algorithm built in the 2D-to-3D image conversion unit 12. The image output port 13 is electrically connected to the 2D-to-3D image conversion unit 12, and also connected to the 3D display 30, and outputs the stereoscopic image to the 3D display 30 such that the 3D display 30 displays the converted stereoscopic images.

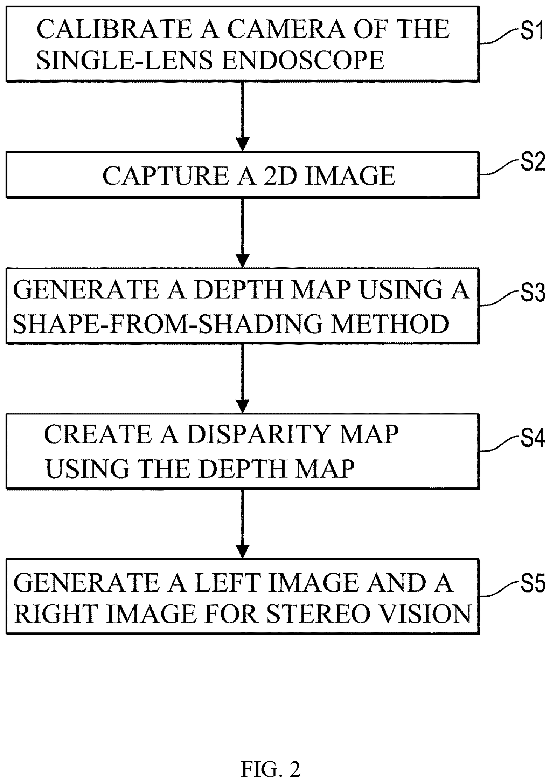

With reference to FIG. 2, a stereoscopic visualization method for endoscope using shape-from-shading algorithm in accordance with the present invention is performed by the 2D-to-3D image conversion unit 12 to convert the 2D images from the monoscopic endoscope 20 into the stereoscopic images, and includes the following steps.

Step S1: Calibrating a camera of the monoscopic endoscope. With reference to "Image processing, analysis and machine vision, 2nd edition, vol. 68, PWS, 1998, pp. 448-457", a camera calibration method is used to calculate intrinsic parameters of the camera of the monoscopic endoscope. The camera calibration method estimates a camera posture by rotating and displacing a calibration template, and solves a nonlinear equation to obtain the intrinsic parameters and extrinsic parameters.

Step S2: Capturing a 2D image. An image-capturing device is used to acquire a 2D image from the camera of the monoscopic endoscope. The image-capturing device may have a resolution being standard definition (SD) or high definition (HD). The camera of the monoscopic endoscope may have a 30 degree lens or a wide angle lens.

Step S3: Generating a depth map using shape-from-shading method. With reference to "Metric depth recovery from monocular images using shape-from-shading and specularities, Visentini-Scarzanella et al. 2012 IEEE Internal Conference on Image Processing", a shape-from-shading algorithm is employed to calculate lighting information and shading information of the 2D image generated from a light source. Then use an iterative approach to solve equations involving gradient variation of pixel information in the 2D image, and combine information associated with an illumination direction and a position of the light source to calculate a depth map of the pixels in the 2D image relative to the light source. An illumination position estimation of the light source disclosed in "Danail Stoyanov et al., 2009 IEEE/RSJ International Conference on Intelligent Robots and System (IROS), Illumination position estimation for 3D soft tissue reconstruction in robotic minimally invasive surgery" is provided to enhance accuracy in determining position of a light source. The pixel information of each pixel in the 2D image includes a pixel intensity value, the illumination direction and the natural logarithm of coordinates of the pixel. Fast sweeping methods disclosed in "Chiu-Yen Kao et al. SIAM J., Numerical Analysis 2005, Fast sweeping methods for static Hamilton-Jacobi equation" and parallel computation can be applied to speed up the iterative process.

The shape-from-shading algorithm can be described by calculation of light distribution of a light source in the following.

Assume that a camera is located at C($\alpha$, $\beta$, y), which can be pre-determined with the illumination position estimation. Given a set of coordinates of each pixel $\mathbf{x} = (x,y)$ in the 2D image, a surface normal $\mathbf{n}$ and a light vector $\mathbf{1}$ at a 3D point M corresponding to the pixel $\mathbf{x}$ can be represented as:

$$\mathbf{n} = \left( u_x, u_y, -\frac{(x+\alpha)u_x + (y+\beta)u_y + u(x)}{f + \gamma} \right)$$

$$\mathbf{1} = (x + \alpha, y + \beta, f + \gamma)$$

where $u(x)$ is the depth at point $\mathbf{x}$ and $u_x$, $u_y$ are the spatial derivatives.

Hence, an image irradiance equation can be expressed as follows in terms of the proposed parametrizations of **l** and **n** without ignoring the distance attenuation term between the light source and surface reflection to solve a conventional Lambertian SFS (Shape-from-shading) model.

$$I(\mathrm{x}) = \rho \frac{\mathbf{l} \cdot \mathbf{n}}{\gamma^2}$$

where $\rho$ is a surface albedo.

After the substitution $v = \ln u$ is performed, a Hamiltonian, which is known as a spatial transformation between the position of the camera and the light source, can be obtained as follows:

$$H(x, \nabla v) = I(\mathrm{x}) \frac{1}{\rho} \sqrt{(v_x^2 + v_y^2 + \mathcal{J}(\mathrm{x}, \nabla v)^2} \cdot Q(\mathrm{x})^{\frac{3}{2}}$$

where

$$\begin{cases} \mathcal{J}(\mathrm{x}, \nabla v) = \dfrac{v_x(x + \alpha) + v_y(y + \beta) + 1}{f + \gamma} \\ Q(\mathrm{x}) = (x + \alpha)^2 + (y + \beta)^2 + (f + \gamma)^2 \end{cases}$$

The depth map of the image caused by light distribution can thus be generated after iterations of calculation of the foregoing equations. As being almost the same, the light vector and the camera position vector can be simplified to be the same vector.

Step S4: Creating a disparity map using the depth map. The depth map is composed of a gray-level image containing information relating to the distance of scene objects on the 2D image from a viewpoint. During the course of converting the depth map into a 3D stereo image pair, a disparity map is generated. Disparity values in the disparity map are inversely proportional to the corresponding pixel intensity values of the depth maps but are proportional to a focal length of a camera of the monoscopic endoscope and an interorbital width of a viewer.

Step S5: Generate a left image and a right image for stereo vision. The disparity map acquired during the course of converting the depth map into the 3D stereo image pair is used for generation of a left eye image and a right eye image. Each disparity value of the disparity map represents a distance between two corresponding points in the left eye image and the right eye image for generation of the left eye image and the right eye image associated with the 3D stereo image pair. The generated left eye image and right eye image can be further processed for various 3D display formats, such as side-by-side, interlaced and other 3D display formats, for corresponding 3D displays to display.

As can be seen from the foregoing description, the depth information can be calculated from the 2D image by using the shape-from-shading algorithm. After generation of the depth map, the 2D images can be combined with the depth maps to generate corresponding stereoscopic images without either replacing the conventional monoscopic endoscope with a dual-lens endoscope or altering the hardware structure of the conventional monoscopic endoscope. Accordingly, the issues arising from the conventional monoscopic endoscope providing no 3D stereo images and the costly dual-lens endoscope can be resolved.

Even though numerous characteristics and advantages of the present invention have been set forth in the foregoing description, together with details of the structure and function of the invention, the disclosure is illustrative only. Changes may be made in detail, especially in matters of shape, size, and arrangement of parts within the principles of the invention to the full extent indicated by the broad general meaning of the terms in which the appended claims are expressed.

**Claims**

1. A stereoscopic visualization system for endoscope using shape-from-shading algorithm, comprising:

    a monoscopic endoscope (20) capturing a two-dimensional (2D) image;

a three-dimensional (3D) display (30); and
an image conversion device (10) connected between the monoscopic endoscope (20) and the 3D display (30), and having:

an input port for endoscope (11) connected to the monoscopic endoscope (20) to receive the 2D image from the monoscopic endoscope (20);
a 2D-to-3D conversion unit (12);
an image output port (13) connected with the 2D-to-3D image conversion unit and the three-dimensional display (30) to receive the stereo image and display the stereo image on the 3D display (30);
wherein the 2D-to-3D conversion unit is configured to apply a shape-from-shading algorithm adapted to calculate a light direction of a light source and a camera position for the 2D image, and to calculate a depth map based upon information of light distribution and shading of the 2D image, and to apply a depth image based rendering algorithm to convert the 2D image to a stereoscopic image with the depth map, wherein a disparity map is created by using the depth map, disparity values in the disparity map are inversely proportional to the corresponding pixel intensity values of the depth maps and are proportional to a focal length of the monoscopic endoscope (20) and an interorbital width of the 3D display (30), and the stereoscopic image is generated by using the disparity map; and
wherein the shape-from-shading algorithm combines the light direction and an iterative approach to solve equations involving a gradient variation of pixel intensity values in the 2D image.

2. A stereoscopic visualization method for endoscope using shape-from-shading algorithm, comprising steps of:

acquiring a 2D image from a monoscopic endoscope with illumination from a light source;
calculating a light direction and a camera position for the 2D image;
generating a depth map of the 2D image using shape-from-shading algorithm, wherein the shape-from-shading algorithm combines the light direction and an iterative approach to solve equations involving a gradient variation of pixel intensity values in the 2D image;
using the depth map to create a disparity map, wherein disparity values in the disparity map are inversely proportional to the corresponding pixel intensity values of the depth maps and are proportional to a focal length of the monoscopic endoscope and an interorbital width of the 3D display;
generating a stereoscopic image by combining the depth map and the 2D image, wherein the stereoscopic image is generated by using the disparity map.

3. The stereoscopic visualization method of claim 2, wherein the shape-from-shading algorithm is based on calculation of light distribution of a light source as follows:
assume that a camera is located at $C(\alpha, \beta, y)$, which can be pre-determined with a illumination position estimation, a set of coordinates of each pixel $\mathbf{x} = (x,y)$ in the 2D image, a surface normal $\mathbf{n}$ and a light vector $\mathbf{l}$ at a 3D point corresponding to the pixel $\mathbf{x}$ of the 2D image are represented as:

$$\mathbf{n} = \left( u_x, u_y, -\frac{(x+\alpha)u_x + (y+\beta)u_y + u(x)}{f + \gamma} \right)$$

$$\mathbf{l} = (x+\alpha, y+\beta, f+\gamma)$$

where $u(x)$ is a depth at point $\mathbf{x}$ and $u_x, u_y$ are spatial derivatives;
an image irradiance equation is expressed as follows in terms of the light vector $\mathbf{l}$ and the surface normal $\mathbf{n}$ without ignoring distance attenuation between the light source and surface reflection to solve a Lambertian SFS (Shape-from-shading) model:

$$I(\mathbf{x}) = \rho \frac{\mathbf{l} \cdot \mathbf{n}}{\gamma^l}$$

where $\rho$ is a surface albedo;

after the substitution $v = \ln u$ is performed, a Hamiltonian, which is known as a spatial transformation between the position of the camera and the light source, is obtained as follows:

$$H(x, \nabla v) = I(x)\frac{1}{\rho}\sqrt{(v_x^2 + v_y^2 + \mathcal{J}(x, \nabla v)^2} \cdot Q(x)^{\frac{3}{2}}$$

where

$$\begin{cases} \mathcal{J}(x, \nabla v) = \dfrac{v_x(x + \alpha) + v_y(y + \beta) + 1}{f + \gamma} \\ Q(x) = (x + \alpha)^2 + (y + \beta)^2 + (f + \gamma)^1 \end{cases}$$

the depth map of the 2D image caused by light distribution is generated after iterations of calculation of the foregoing equations, and the light vector and the camera position vector are simplified to be the same vector.

4. The stereoscopic visualization method of claim 2, wherein the stereoscopic image is generated according to the depth image based rendering algorithm to provide different views of the 2D image with the 2D image and the depth map.

**Patentansprüche**

1. Stereoskopisches Visualisierungssystem für ein Endoskop unter Verwendung des Shape-from-Shading-Algorithmus, Folgendes umfassend:

ein ein zweidimensionales (2D-) Bild erfassendes monoskopisches Endoskop (20);
ein dreidimensionales (3D-) Display (30); und
eine zwischen dem monoskopischen Endoskop (20) und dem 3D-Display (30) angeschlossene Bildumwandlungsvorrichtung (10), welche Folgendes umfasst:

einen Eingangsport (11) für ein Endoskop, welcher mit dem monoskopischen Endoskop (20) verbunden ist, um das 2D-Bild vom monoskopischen Endoskop (20) zu empfangen;
eine 2D-in-3D-Umwandlungseinheit (12);
einen Bildausgabeport (13), welcher mit der 2D-in-3D-Umwandlungseinheit und dem dreidimensionalen Display (30) verbunden ist, um das Stereobild zu empfangen und das Stereobild auf dem 3D-Display (30) anzuzeigen;
wobei die 2D-in-3D-Umwandlungseinheit derart konfiguriert ist,
dass sie einen Shape-from-Shading-Algorithmus anwendet, welcher dazu geeignet ist, eine Lichtrichtung einer Lichtquelle und eine Kameraposition für das 2D-Bild zu berechnen, und basierend auf den Informationen über die Lichtverteilung und die Schattierung des 2D-Bildes eine Tiefenkarte zu berechnen, und derart konfiguriert ist, dass sie einen auf einem Tiefenbild basierenden Rendering-Algorithmus einsetzt, um das 2D-Bild mit der Tiefenkarte in ein stereoskopisches Bild umzuwandeln, wobei durch das Verwenden der Tiefenkarte eine Disparitätskarte erstellt wird, wobei Disparitätswerte auf der Disparitätskarte umgekehrt proportional zu den entsprechenden Pixelintensitätswerten der Tiefenkarten und proportional zu einer Brennweite des monoskopischen Endoskops (20) und einer interorbitalen Breite des 3D-Displays (30) sind, und wobei das stereoskopische Bild durch das Verwenden der Disparitätskarte erzeugt wird; und

wobei der Shape-from-Shading-Algorithmus die Lichtrichtung und einen iterativen Ansatz miteinander kombiniert, um Gleichungen zu lösen, welche eine Gradientenvariation von Pixelintensitätswerten in dem 2D-Bild umfassen.

2. Stereoskopisches Visualisierungsverfahren für ein Endoskop unter Verwendung des Shape-from-Shading-Algorithmus, folgende Schritte umfassend:

Erhalten eines 2D-Bildes von einem monoskopischen Endoskop mittels Beleuchtung durch eine Lichtquelle;
Berechnen einer Lichtrichtung und einer Kameraposition für das 2D-Bild;
Erstellen einer Tiefenkarte des 2D-Bildes unter Verwendung des Shape-from-Shading-Algorithmus, wobei der Shape-from-Shading-Algorithmus die Lichtrichtung und einen iterativen Ansatz miteinander kombiniert, um Gleichungen zu lösen, welche eine Gradientenvariation von Pixelintensitätswerten in dem 2D-Bild umfassen;
Verwenden der Tiefenkarte, um eine Disparitätskarte zu erstellen, wobei Disparitätswerte auf der Disparitätskarte umgekehrt proportional zu den entsprechenden Pixelintensitätswerten der Tiefenkarten und proportional zu einer Brennweite des monoskopischen Endoskops und einer interorbitalen Breite des 3D-Displays sind;
Erzeugen eines stereoskopischen Bildes durch das Kombinieren der Tiefenkarte mit dem 2D-Bild, wobei das stereoskopische Bild durch das Verwenden der Disparitätskarte erzeugt wird.

3. Stereoskopisches Visualisierungsverfahren nach Anspruch 2, wobei der Shape-from-Shading-Algorithmus auf der folgenden Berechnung der Lichtverteilung einer Lichtquelle basiert:

unter der Annahme, dass sich eine Kamera an Punkt C($\alpha$, $\beta$, $\gamma$) befindet, welcher mittels einer Schätzung der Beleuchtungsposition vorherbestimmt werden kann, werden ein Koordinatensatz zu jedem Pixel $\mathbf{x} = (x,y)$ in dem 2D-Bild, eine Flächennormale $\mathbf{n}$ und ein Lichtvektor $\mathbf{l}$ an einem dem Pixel x des 2D-Bildes entsprechenden 3D-Punkt wie folgt dargestellt:

$$\mathbf{n} = \left( u_x, u_y, -\frac{(x+\alpha)u_x + (y+\beta)u_y + u(x)}{f+\gamma} \right)$$

$$\mathbf{l} = (x+\alpha, y+\beta, f+\gamma)$$

wobei $u(x)$ eine Tiefe an Punkt $\mathbf{x}$ und $u_x, u_y$ räumliche Ableitungen sind;
eine Gleichung zur Bildbestrahlungsstärke wird, ohne eine Distanzdämpfung zwischen der Lichtquelle und einer Oberflächenreflexion zu vernachlässigen, in Form des Lichtvektors $\mathbf{l}$ und der Flächennormalen $\mathbf{n}$ wie folgt ausgedrückt, um ein Lambertsches SFS (Shape-from-Shading) Modell zu lösen:

$$I(\mathbf{x}) = \rho \frac{\mathbf{l} \cdot \mathbf{n}}{\gamma^1}$$

wobei $\rho$ ein Oberflächenrückstrahlvermögen ist;
nachdem die Substitution $v = \ln u$ durchgeführt wurde, wird eine Hamilton-Funktion, welche als räumliche Transformation zwischen der Kameraposition und der Lichtquelle bekannt ist, wie folgt erhalten:

$$H(x, \nabla v) = I(x) \frac{1}{\rho} \sqrt{(v_x^2 + v_y^2 + J(x, \nabla v)^2} \cdot Q(x)^{\frac{3}{2}}$$

wobei

$$\begin{cases} J(\mathbf{x}, \nabla v) = \dfrac{v_x(x+\alpha) + v_y(y+\beta) + 1}{f+\gamma} \\ Q(x) = (x+\alpha)^2 + (y+\beta)^2 + (f+\gamma)^1 \end{cases} ;$$

die Tiefenkarte des durch Lichtverteilung entstandenen 2-D-Bildes wird nach Iterationen der Berechnung der vorhergehenden Gleichungen erzeugt, und der Lichtvektor und die Kameraposition sind derart vereinfacht, dass sie den gleichen Vektor bilden.

4. Stereoskopisches Visualisierungsverfahren nach Anspruch 2, wobei das stereoskopische Bild nach dem auf dem Tiefenbild basierenden Rendering-Algorithmus erzeugt wird, um verschiedene Ansichten des 2D-Bildes mit dem 2D-Bild und der Tiefenkarte bereitzustellen.

**Revendications**

1. Système de visualisation stéréoscopique pour un endoscope utilisant un algorithme de *shape from shading,* comprenant :

   un endoscope monoscopique (20) capturant une image bidimensionnelle (2D) ;
   un écran tridimensionnel (3D) (30) ; et
   un dispositif de conversion d'image (10) connecté entre l'endoscope monoscopique (20) et l'écran 3D (30), et ayant :

   un port d'entrée (11) pour un endoscope connecté à l'endoscope monoscopique (20) pour recevoir l'image 2D de l'endoscope monoscopique (20) ;
   une unité de conversion 2D à 3D (12) ;
   un port de sortie d'image (13) connecté avec l'unité de conversion 2D à 3D et l'écran tridimensionnel (30) pour recevoir l'image stéréo et pour afficher l'image stéréo sur l'écran 3D (30) ;
   l'unité de conversion 2D à 3D étant configurée pour appliquer un algorithme de *shape from shading* adapté pour calculer une direction de lumière d'une source de lumière et une position de la caméra pour l'image 2D, et pour calculer une carte de profondeur basée sur des informations sur la distribution de lumière et l'ombrage de l'image 2D, et
   étant configurée pour appliquer un algorithme de rendu basé sur une image de profondeur pour convertir l'image 2D avec la carte de profondeur en une image stéréoscopique, une carte de disparité étant créée en utilisant la carte de profondeur, des valeurs de disparité dans la carte de disparité étant inversement proportionnelles aux valeurs d'intensité de pixel correspondantes des cartes de profondeur et proportionnelles à une distance focale de l'endoscope monoscopique (20) et une largeur interorbitale de l'écran 3D (30), et l'image stéréoscopique étant générée en utilisant la carte de disparité ; et
   l'algorithme de *shape from shading* combinant la direction de lumière et une démarche itérative pour résoudre des équations comprenant une variation du gradient des valeurs d'intensité de pixel dans l'image 2D.

2. Procédé de visualisation stéréoscopique pour un endoscope utilisant un algorithme de *shape from shading,* comprenant des étapes suivantes :

   acquérir une image 2D d'un endoscope monoscopique à l'aide de l'illumination par une source de lumière ;
   calculer une direction de lumière et une position de la caméra pour l'image 2D ;
   générer une carte de profondeur de l'image 2D en utilisant un algorithme de *shape from shading,* l'algorithme de *shape from shading* combinant la direction de lumière et une démarche itérative pour résoudre des équations comprenant une variation du gradient des valeurs d'intensité de pixel dans l'image 2D ;
   utiliser une carte de profondeur pour créer une carte de disparité, des valeurs de disparité dans la carte de disparité étant inversement proportionnelles aux valeurs d'intensité de pixel correspondantes des cartes de profondeur et proportionnelles à une distance focale de l'endoscope monoscopique et une largeur interorbitale de l'écran 3D ;
   générer une image stéréoscopique en combinant la carte de profondeur avec l'image 2D, l'image stéréoscopique étant générée en utilisant la carte de disparité.

3. Procédé de visualisation stéréoscopique selon la revendication 2, l'algorithme de *shape from shading* étant basé sur le calcul de la distribution de lumière d'une source de lumière comme suit :
   en supposant qu'une caméra soit située à un point $C(\alpha, \beta, \gamma)$, qui peut être prédéterminé à l'aide d'une estimation de la position d'illumination, un ensemble de coordonnées de chaque pixel $x = (x,y)$ dans l'image 2D, une normale à la surface **n** et un vecteur de lumière **l** à un point 3D correspondant au pixel **x** de l'image 2D sont représentés par :

$$\mathbf{n} = \left( u_x, u_y, -\frac{(x + \alpha)u_x + (y + \beta)u_y + u(x)}{f + \gamma} \right)$$

$$\mathbf{l} = (x + \alpha, y + \beta, f + \gamma)$$

$u(x)$ étant une profondeur au point **x** et $u_x, u_y$ étant des dérivés spatiaux ;
une équation d'irradiance d'image est exprimée comme suit en termes du vecteur de lumière **l** et de la normale à la surface **n,** sans ignorer une atténuation de la distance entre la source de lumière et une réflexion de surface, pour résoudre un modèle SFS (*shape from shading*) lambertien :

$$I(\mathbf{x}) = \rho \frac{\mathbf{l} \cdot \mathbf{n}}{\gamma^2}$$

$\rho$ étant un albédo de la surface ;
après l'exécution de la substitution $v = \ln u$ , un opérateur hamiltonien, qui est connu comme transformation spatiale entre la position de la caméra et la source de lumière, est obtenu comme suit :

$$H(x, \nabla v) = I(\mathbf{x}) \frac{1}{\rho} \sqrt{(v_x^2 + v_y^2 + \mathcal{J}(\mathbf{x}, \nabla v)^2} \cdot Q(\mathbf{x})^{\frac{3}{2}}$$

dans lequel

$$
\begin{cases}
\mathcal{J}(\mathbf{x}, \nabla v) = \dfrac{v_x(x + \alpha) + v_y(y + \beta) + 1}{f + \gamma} \\
Q(\mathbf{x}) = (x + \alpha)^2 + (y + \beta)^2 + (f + \gamma)^2
\end{cases} ;
$$

la carte de profondeur de l'image 2D causée par la distribution de lumière est générée après des itérations de calcul des équations précédentes, et le vecteur de lumière et la position de la caméra sont simplifiés pour être le même vecteur.

4. Procédé de visualisation stéréoscopique selon la revendication 2, l'image stéréoscopique étant générée selon l'algorithme de rendu basé sur une image de profondeur pour fournir des vues différentes de l'image 2D avec l'image 2D et la carte de profondeur.

IMAGE CONVERSION DEVICE

MONOSCOPIC ENDOSCOPE

3D DISPLAY

FIG. 1

CALIBRATE A CAMERA OF THE
SINGLE-LENS ENDOSCOPE — S1

CAPTURE A 2D IMAGE — S2

GENERATE A DEPTH MAP USING A
SHAPE-FROM-SHADING METHOD — S3

CREATE A DISPARITY MAP
USING THE DEPTH MAP — S4

GENERATE A LEFT IMAGE AND A
RIGHT IMAGE FOR STEREO VISION — S5

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2014198976 A

### Non-patent literature cited in the description

- Image processing, analysis and machine vision. PWS. 1998, vol. 68, 448-457
- **VISENTINI-SCARZANELLA et al.** Metric depth recovery from monocular images using shape-from-shading and specularities. *IEEE Internal Conference on Image Processing,* 2012
- **DANAIL STOYANOV et al.** *IEEE/RSJ International Conference on Intelligent Robots and System (IROS),* 2009
- **CHIU-YEN KAO et al.** *SIAM J., Numerical Analysis,* 2005